(19)

Europäisches
Patentamt
European
Patent Office
Office européen
des brevets

(11) **EP 3 620 127 A1**

(12) **EUROPEAN PATENT APPLICATION**

(43) Date of publication:
**11.03.2020 Bulletin 2020/11**

(21) Application number: **19195072.4**

(22) Date of filing: **03.09.2019**

(51) Int Cl.:
*A61B 34/20* (2016.01)          *A61B 5/06* (2006.01)
*G01R 33/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.09.2018  US 201816120718**

(71) Applicant: **Biosense Webster (Israel) Ltd.
Yokneam, 2066717 (IL)**

(72) Inventors:
• **GLINER, Vadim
  2066717 Yokneam (IL)**
• **GOVARI, Assaf
  2066717 Yokneam (IL)**

(74) Representative: **Small, Gary James
  Carpmaels & Ransford LLP
  One Southampton Row
  London WC1B 5HA (GB)**

(54)     **SINGLE AXIS SENSOR (SAS) WITH HALL SENSOR USING EXTERNAL MAGNET**

(57)     A catheter-based tracking system includes one or more field generators and a processor. The one or more field generators, located adjacent to a Magnetic Resonance Imagining (MRI) system, are configured to apply Alternating Current (AC) magnetic fields. The processor is configured to receive signals, which are produced responsively to the AC magnetic fields in a single axis sensor (SAS) that is fitted at a distal end of a catheter inserted into an organ of a patient, to calculate, based on the signals received from the SAS, a direction of the distal end, to receive from a Hall effect sensor that is fitted at the distal end of the catheter a sensed component of a Direct Current (DC) magnetic field of the magnetic resonance imagining (MRI) system, and, based on the calculated direction and the sensed component of the DC magnetic field, to calculate a roll-angle of the distal end inside the organ.

FIG. 2A

**Description**

**FIELD OF THE INVENTION**

The present invention relates generally to tracking of intrabody probes, and particularly to magnetic position and orientation sensors in catheter-based tracking systems.

**BACKGROUND OF THE INVENTION**

Various techniques for magnetically tracking a position and/or an orientation of intra-body probes were previously proposed. For example, U.S. Patent Application Publication 2010/0210939 describes a surgical navigation system for tracking an instrument relative to a patient. The system can track a portion of the patient, an instrument, and/or both relative to image data, a coordinate system, an atlas, a morphed atlas, or combinations thereof. The system can include a tracking device on the instrument to provide six degree of freedom information regarding the location of the instrument. In an embodiment, a location sensor in the device includes two coils are which placed in an angle relative to one another, such as an orthogonal angle. The sensor may use Hall sensors alternatively to coils.

As another example, U.S. Patent Application Publication 2016/0278746 describes a system and method that pertains to an MR-guided breast biopsy procedure, specifically as to real-time tracking and navigation of a biopsy device. More particularly, the system utilizes a diagnostic imaging modality such as magnetic resonance imaging (MRI) to locate lesions in a human breast while utilizing an inertial measurement unit to track advancement of a biopsy device in real-time. One tracking approach is based on a set of 3-axis Hall-effect-gyroscope-accelerometer sensors: respectively, sensors/transducers yield varying output voltages in response to the different magnetic fields sensed, as well as for different accelerations and angular velocities.

U.S. Patent Application Publication 2012/0143127 describes a variable magnet system for manipulating a magnetic catheter. In one embodiment, a cluster of electromagnets is configured to generate a desired magnetic field. In one embodiment, one or more poles of the cluster are moveable with respect to other poles in the cluster to allow shaping of the magnetic field. In one embodiment, one or more magnetic poles can be extended or retracted to shape the magnetic field. In one embodiment, the electromagnets can be positioned to generate magnetic fields that exert a desired torque and/or movement force on the catheter. In one embodiment, a magnetic field source is used to create a magnetic field of sufficient strength and orientation to move a magnetically-responsive catheter tip in a desired direction by a desired amount.

U.S. Patent Application Publication 2003/0006759 describes a position sensor for a medical device that comprises a core made of a high permeable material such as Wiegand effect material comprising a mixture of cobalt, vanadium, and iron. The position sensor has an outer diameter of approximately 0.4 mm and is used in a medical device having an outer diameter of approximately 0.67 mm.

**SUMMARY OF THE INVENTION**

An embodiment of the present invention provides a catheter-based tracking system including one or more field generators and a processor. The one or more field generators, which are located adjacent to a Magnetic Resonance Imagining (MRI) system, are configured to apply one or more Alternating Current (AC) magnetic fields. The processor is configured to receive signals, which are produced responsively to the AC magnetic fields in a single axis sensor (SAS) that is fitted at a distal end of a catheter inserted into an organ of a patient, and, based on the signals received from the SAS, calculate a direction of the distal end. The processor is further configured to receive, from a Hall effect sensor that is fitted at the distal end of the catheter, a sensed component of a Direct Current (DC) magnetic field of the magnetic resonance imagining (MRI) system, and, based on the calculated direction and the sensed component of the DC magnetic field, calculate a roll-angle of the distal end inside the organ.

In some embodiments, the processor is further configured to calculate a position of the distal end based on the signals received from the SAS.

In some embodiments, the processor is further configured to present the roll angle of the distal end in a coordinate system of the catheter-based tracking system.

There is additionally provided, in accordance with an embodiment of the present invention, a method, including applying one or more Alternating Current (AC) magnetic fields using one or more field generators, which are located adjacent to a Magnetic Resonance Imagining (MRI) system. Signals are received, which are produced responsively to the AC magnetic fields in a single axis sensor (SAS) that is fitted at a distal end of a catheter inserted into an organ of a patient. A direction of the distal end is calculated based on the signals received from the SAS. A sensed component of a Direct Current (DC) magnetic field of the magnetic resonance imagining (MRI) system is received from a Hall effect sensor that is fitted at the distal end of the catheter. Based on the calculated direction and the sensed component of the DC magnetic field, a roll-angle of the distal end inside the organ is calculated.

There is additionally provided, in accordance with an embodiment of the present invention, a magnetic sensor including a single axis sensor (SAS) and a Hall effect sensor. The single axis sensor (SAS) is configured to, in response to one or more Alternating Current (AC) magnetic fields, generate signals indicative of a direction of a single axis of the SAS in a given coordinate system. The Hall effect sensor, which is configured to, in response to a Direct Current (DC) magnetic field, generate a signal indicative, in the given coordinate system, of a roll-angle of the SAS about the single axis.

The present invention will be more fully understood from the following detailed description of the embodiments thereof, taken together with the drawings in which:

## BRIEF DESCRIPTION OF THE DRAWINGS

Fig. 1 is a schematic, pictorial illustration of a catheter-based magnetic position-tracking system, in accordance with an embodiment of the present invention;

Figs. 2A and 2B are side views of a distal end of a catheter comprising a Hall effect sensor oriented in a parallel roll-angle and an orthogonal roll-angle, respectively, in accordance with an embodiment of the present invention;

Fig. 3 is a graph of normalized Hall voltage as a function of roll-angle $\alpha$, in accordance with an embodiment of the present invention; and

Fig. 4 is a flow chart that schematically illustrates a method for estimating the roll-angle of a catheter using a Hall effect sensor, in accordance with an embodiment of the present invention.

## DETAILED DESCRIPTION OF EMBODIMENTS

OVERVIEW

Embodiments of the present invention that are described hereinafter provide a magnetic catheter-based tracking system that is configured to utilize a presence of a large constant magnetic field, such as of a magnet a magnetic resonance imaging (MRI) system, to measure a roll angle of a distal end of the catheter. The MRI system is available in many cases since it is being used for imaging an organ of a patient to which the catheter is inserted.

In some embodiments, a processor of the magnetic tracking system registers the axis (direction) of the constant (Direct Current - DC) magnetic field of the MRI system, with a coordinate system of the magnetic catheter-based tracking system. For example, a component of the MRI magnetic field that affects the roll-angle indicative signals is projected by the processor onto the axes of the coordinate system of the catheter-based tracking system. With this additional projection, a full description of the catheter distal end position, direction, and roll-angle, is available in the coordinate system of the catheter-based tracking system.

In some embodiments, a miniature magnetic sensor is provided, which is fitted at the distal end of the catheter. The magnetic sensor is capable of generating signals indicative of the position, direction, and roll-angle of the distal end of the catheter inside an organ of a patient in a given coordinate system, such as the coordinate system of the catheter-based tracking system. The disclosed miniature magnetic sensor comprises a single-axis-sensor (SAS - typically a coil) and a Hall effect sensor that are both fitted at the catheter distal end, as described below.

In some embodiments, the SAS generates the position and direction signals in response to one or more alternating (Alternating Current - AC) magnetic fields of the magnetic catheter-based tracking system. The Hall effect sensor generates a roll-angle indicative signal in response to a constant magnetic field that is induced by the additional external magnet of the MRI system.

In the description hereinafter, a roll-angle of the catheter distal end is defined as an amount of rotation of the catheter distal end about a longitudinal axis of symmetry of the distal end. The roll-angle can be used, for example, to improve the accuracy of electroanatomical maps produced by electrophysiological (EP) mapping catheters, and/or of ablation procedures, by enabling, for example, controlling the roll of a catheter that has asymmetric electrodes for EP sensing or ablation. The roll-angle indicative signal comprises a Hall voltage that varies as the catheter rolls over its longitudinal axis of symmetry, as described below.

In some embodiments, the SAS comprises a miniaturized coil sensor, and the disclosed miniature magnetic sensor structure combines the Hall effect sensor with the miniaturized coil sensor. The magnetic sensor is also named hereinafter "position, direction, and roll-angle (PDR) sensor." In some embodiments, the PDR sensor is made of a single coil that is wound to enclose the Hall effect sensor. A direct (DC) electric current is applied to the Hall effect sensor, and when the distal end is placed in the DC magnetic field of the MRI, a Hall voltage is generated at the terminals of the Hall effect sensor.

The maximal amplitude of the Hall voltage depends on the direction of the distal end in space, via the projection of the external DC magnetic field on that direction. Taking into account the known (e.g., tracked) direction of the distal end, and based on the registration of the coordinate systems, a processor calculates a Hall voltage that is uniquely indicative of the roll-angle. For example, in an embodiment, the processor calculates an instantaneous direction of the catheter from direction signals received from the SAS, and based on the calculated direction, the measured Hall voltage, and the aforementioned registration of coordinate systems, derives the catheter distal end roll-angle in a coordinate system of the catheter position tracking system.

Typically, miniature Hall effect sensors are made of a specific semiconductor, such as GaAs. Generally, however, under a strong DC magnetic field, such as of an MRI system, a measurable Hall voltage can be readily generated by many types of conductors and semiconductors. In an embodiment, one or more metallic electrodes that are disposed at the distal end of the catheter are utilized as Hall sensors, and terminals are connected to the electrodes to output generated Hall voltage.

Note that bulky sensors, such as multi-coil sensors, may alternatively be used for the simultaneous measurement of position, direction, and roll-angle. Such sensors, however, require an increased diameter of the catheter distal end and may thus limit maneuverability in the body. The disclosed miniature Hall sensor, on the other hand, which has sub-millimeter dimensions, can be fitted in a narrow diameter distal end of a catheter. The disclosed PDR sensor, thus, adds signals indicative of its roll-angle on top of the position and direction signals provided by a legacy miniaturized SAS sensor, while presenting the same, or a similar, physical form factor of the SAS.

The disclosed roll-angle tracking technique, which utilizes a large DC magnetic field of an MRI system that is available anyhow, to determine roll-angle using the disclosed miniature PDR sensor, can improve catheter accessibility and tracking accuracy, and therefore also improve the overall quality of diagnostic and therapeutic catheterization procedures.

SYSTEM DESCRIPTION

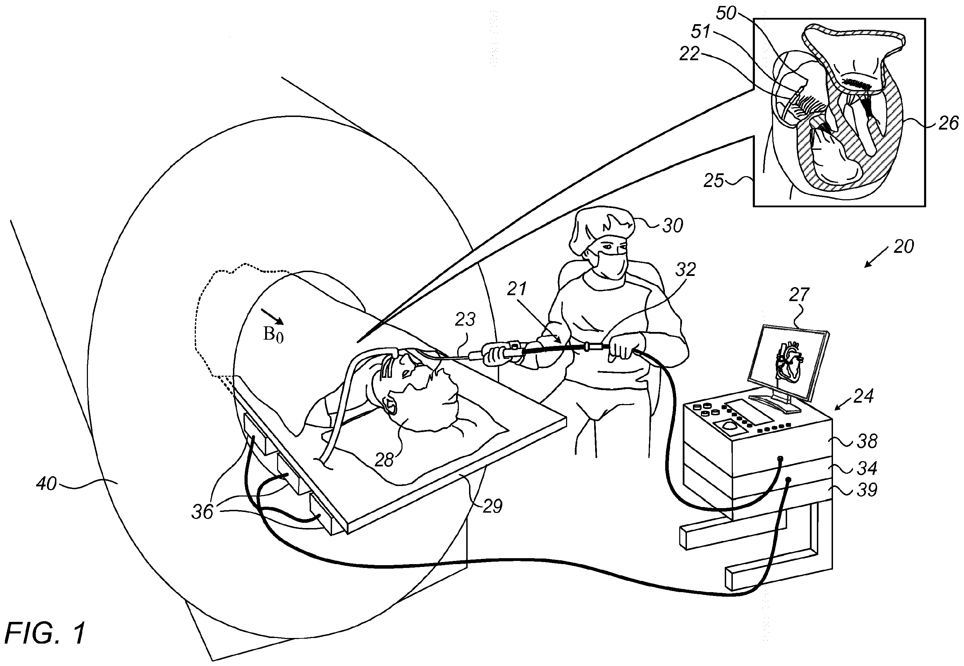

Fig. 1 is a schematic, pictorial illustration of a magnetic catheter-based tracking system 20, in accordance with an embodiment of the present invention. System 20 comprises a catheter 21, having a distal end 22 that is navigated by a physician 30 into a heart 26 of a patient 28 via the vascular system. The catheter can be used for ablation, EP sensing, or any other medical procedure. In the pictured example, physician 30 inserts distal end 22 through a sheath 23, while manipulating distal end 22 using a manipulator 32 near the proximal end of the catheter. As shown in an inset 25, distal end 22 comprises a PDR sensor 51 which is contained within distal end 22, and an ablation catheter 50.

In the embodiments described herein, catheter 21 is used for ablation of tissue in heart 26. Although the pictured embodiment relates specifically to the use of an ablation catheter 50 for ablation of heart tissue, the elements of system 20 and the methods described herein may alternatively be applied to diagnostic applications, such as electrophysiological mapping, using, for example, multi-electrode catheters such as the Pentaray® or the Lasso® catheters (both made by Biosense-Webster, Irvine, California).

The proximal end of catheter 21 is connected to a control console 24. Console 24 comprises a processor 39, typically a general-purpose computer, with suitable front end and interface circuits 38 for receiving signals from catheter 21, as well as for applying energy via catheter 21 to ablate tissue in heart 26 and for controlling the other components of system 20. Console 24 also comprises a driver circuit 34, configured to drive magnetic field generators 36.

During the navigation of distal end 22 in heart 26, console 24 receives position and direction signals from PDR sensor 51 in response to magnetic fields from external field generators 36. Magnetic field generators 36 are placed at known positions external to patient 28, e.g., below a table 29 on which the patient is lying. These position and direction signals are indicative of the position and direction of ablation catheter 50 in a coordinate system of the position tracking system.

PDR sensor 51 further transmits a roll-angle indicative signal to console 24, in response to a large constant magnetic field $B_0$ of an MRI system 40 (e.g., $B_0$ equals 1.5T). $B_0$ defines a longitudinal axis for a coordinate system of the MRI system.

Using the received signals, processor 39 calculates the position, direction, and roll-angle of ablation catheter 50 in the heart and, optionally, presents the tracked position, direction, and roll-angle on a display 27.

The method of position and direction sensing using external magnetic fields is implemented in various medical applications, for example, in the CARTO™ system, produced by Biosense Webster, and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

Processor 39 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

SINGLE AXIS SENSOR (SAS) WITH HALL SENSOR USING EXTERNAL MAGNET

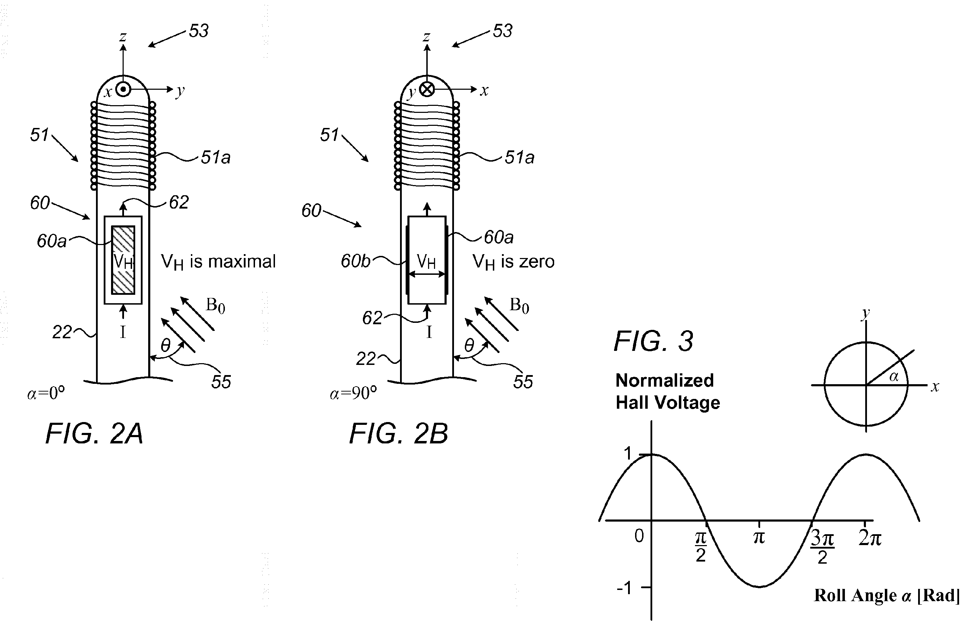

Figs. 2A and 2B are side views of a distal end of a catheter comprising a Hall effect sensor, oriented in a parallel roll-angle and an orthogonal roll-angle, respectively, in accordance with an embodiment of the present invention. In Fig. 2A, $\alpha=0°$, meaning distal end 22, is rolled parallel to an external magnetic field, as described below. In Fig. 2B, distal end 22 is rolled at $\alpha=90°$ relative to the external magnetic field.

As further seen, the MRI magnetic field vector $\mathbf{B_0}$ is directed with an angle 55 (i.e., $\theta$) relative to distal end 22, which in a coordinate system 53 is aligned with its longitudinal axis parallel to the z-axis of coordinate system 53. Roll-angle $\alpha$ of distal end 22 is therefore defined relative to a projected component of magnetic field vector $\mathbf{B_0}$, $\mathbf{B_\theta}=\mathbf{B_0} \cdot Sin(\theta)$.

As can be seen, whatever the roll-angle $\alpha$ of distal end 22, the lines of the magnetic field face the same effective area encompassed by coil 51a of sensor 51 (i.e., SAS 51a). Thus, using only coil 51a, tracking system 20 has no roll-angle indicative signal.

As noted above, adding the disclosed Hall effect sensor 60 provides the required roll-angle indicative signal. Fig. 2A shows an electrical current 62 that flows in the longitudinal direction through Hall effect sensor 60. Electrodes 60a and 60b of a Hall sensor 60 are aligned to sense a resulting Hall voltage perpendicular to a magnetic field component $\mathbf{B_\theta}$ of vector $\mathbf{B_0}$. At roll-angle $\alpha=0°$, exemplified by Fig. 2A, the resulting Hall voltage falling between electrodes 60a and 60b is maximal. In Fig. 2B, distal end 22 is rolled by 90° relative to Fig. 2A, and electrodes 60a and 60b are aligned parallel to magnetic field component $\mathbf{B_\theta}$. In this case the resulting Hall voltage between electrodes 60a and 60b is zero.

As further described below, the Hall voltage is utilized to indicate a continuous roll-angle of distal end 22.

The example illustrations shown in Figs. 2A and 2B are chosen purely for the sake of conceptual clarity. For example, coil 51a, which is shown separated from Hall sensor 60, is typically wound over sensor 60. Other system elements, such as additional sensors and electrodes, are omitted for simplicity.

Fig. 3 is a graph of normalized Hall voltage as a function of roll-angle $\alpha$, in accordance with an embodiment of the present invention. The Hall voltage, $\mathbf{V_H}$, is proportional to the cross product of current 62, $\mathbf{I}$, and magnetic field component $\mathbf{B_\theta}$, i.e.,

$$V_H(\alpha;\theta) = K \cdot I \cdot B_0 \cdot Sin(\theta) \cdot Cos(\alpha)$$

where $\alpha$ is the roll-angle, K is a known constant, and $\mathbf{B_0} \cdot Sin(\theta) \cdot Cos(\alpha)$ is the Hall sensed component of the DC magnetic field $\mathbf{B_0}$.

As shown in Fig. 3, a normalized Hall voltage varies with roll-angle $\alpha$ as a $\mathbf{Cos(\alpha)}$ function. Thus, the measured Hall voltage encodes the roll-angle, e.g., in the form of a cosine function. The Hall voltage provides information regarding the rotation of the SAS around its axis. For example, as noted above, for a certain roll-angle the Hall voltage is maximal. Further rotation of the catheter by 90° causes the Hall voltage to drop to zero. Another rotation by 90° causes the Hall voltage to be maximal but with an opposite polarity. In an embodiment, a processor uses a lookup table comprising roll-angle as a function of Hall voltage, so as to indicate a roll-angle of the distal end in an organ of a patient.

In an embodiment, the roll-angle is indicated in the coordinate system of the catheter tracking system (with which the coordinate system of the MRI system is registered, as described above).

In an optional embodiment, the processor uses slight variations in roll-angle to calculate the slope, $\dfrac{dV_H}{d\alpha}$, of the Hall voltage at a given roll-angle, so as to differentiate between a roll-angle having a same $\mathbf{V_H}$, for example, between a perpendicular (90°) to anti perpendicular (270°) roll-angles of the distal end 22, in which for both $\mathbf{V_H=0}$.

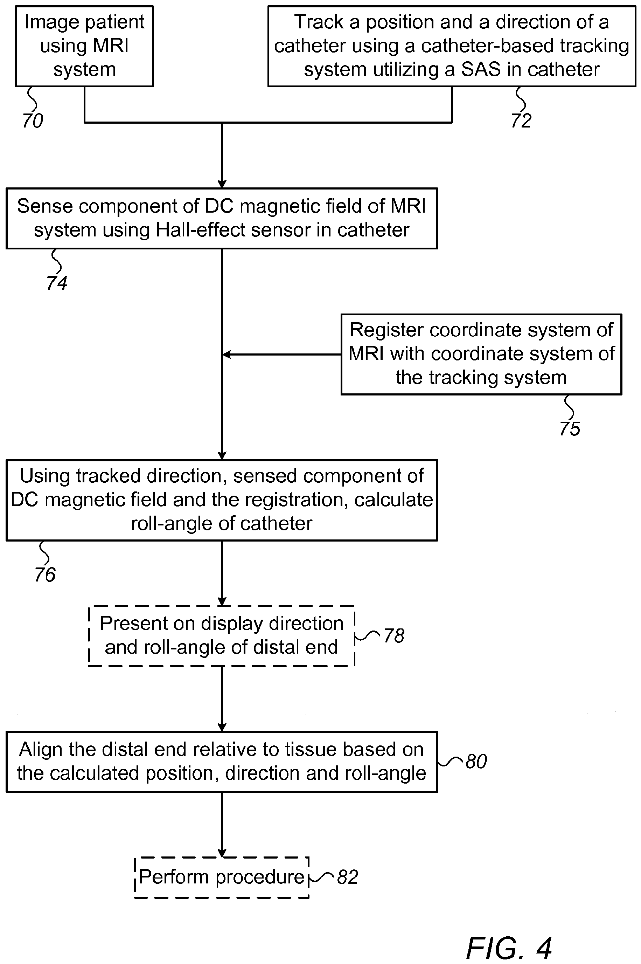

Fig. 4 is a flow chart that schematically illustrates a method for estimating the roll-angle of a catheter using a Hall effect sensor, in accordance with an embodiment of the present invention. The process begins with an MRI system 40 imaging patient 28, at an MRI imaging step 70. In parallel, typically in synchronization with the imaging sequences to avoid electronic noises, catheter-based tracking system 20 tracks a position and a direction of distal end 22 of catheter 21, using modulated magnetic fields that generators 36 produce, at a position and direction tracking step 72. For that, system 20 uses a SAS sensor 51a that is part of PDR sensor 51 that is fitted at distal end 22 of catheter 21. Next, tracking system 20 senses a component of the DC magnetic field of MRI system 40 that includes the roll angle information, $cos(\alpha)$, using Hall-effect sensor 60 that is also part of PDR sensor 51, at a DC magnetic field component sensing step 74. In a coordinate registration step 75, processor 39 registers a direction defined by the DC magnetic field of MRI system 44, $\mathbf{B_0}$, with the coordinate system of magnetic catheter-based tracking system 20.

Then, based on the tracked direction, the Hall sensed component of the DC magnetic field, and the aforementioned registration of coordinate systems, processor 39 calculates a roll-angle of distal end 22, at a roll-angle calculation step 76. In an optional direction and roll-angle presenting step 78, the indications provided by steps 72 and 76 are presented on display 27, on a map of heart 26. Next, physician 30 uses the indications provided by steps 72 and 76 to further spatially align the distal end inside heart 26 of patient 28, at a catheter aligning step 80. For example, physician 30 may roll the distal end so as to be able to ablate target tissue, and perform a procedure, such as an ablation, at an optional perform procedure step 82.

The example flow chart shown in Fig. 4 is chosen purely for the sake of conceptual clarity. In alternative embodiments, additional steps, such as electrophysiological sensing of aberrant cardiac activity may be included.

Although the embodiments described herein mainly address cardiac applications, the methods and systems described herein can also be used in other applications, such as in neurology and otolaryngology and nephrology.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

**Claims**

1.   A catheter-based tracking system, comprising:

   one or more field generators, which are located adjacent to a Magnetic Resonance Imagining (MRI) system and are configured to apply one or more Alternating Current (AC) magnetic fields; and
   a processor, which is configured to:

      receive signals, which are produced responsively to the AC magnetic fields in a single axis sensor (SAS) that is fitted at a distal end of a catheter inserted into an organ of a patient;
      based on the signals received from the SAS, calculate a direction of the distal end;
      receive, from a Hall effect sensor that is fitted at the distal end of the catheter, a sensed component of a Direct Current (DC) magnetic field of the magnetic resonance imagining (MRI) system; and
      based on the calculated direction and the sensed component of the DC magnetic field, calculate a roll-angle of the distal end inside the organ.

2.   The catheter-based tracking system according to claim 1, wherein the processor is further configured to, based on the signals received from the SAS, calculate a position of the distal end.

3.   The catheter-based system according to claim 1, wherein the processor is further configured to present the roll angle of the distal end in a coordinate system of the catheter-based tracking system.

4.   A method, comprising:

   applying one or more Alternating Current (AC) magnetic fields using one or more field generators, which are located adjacent to a Magnetic Resonance Imagining (MRI) system;
   receiving signals, which are produced responsively to the AC magnetic fields in a single axis sensor (SAS) that is fitted at a distal end of a catheter inserted into an organ of a patient;
   based on the signals received from the SAS, calculating a direction of the distal end;
   receiving, from a Hall effect sensor that is fitted at the distal end of the catheter, a sensed component of a Direct Current (DC) magnetic field of the magnetic resonance imagining (MRI) system; and
   based on the calculated direction and the sensed component of the DC magnetic field, calculating a roll-angle of the distal end inside the organ.

5.   The method according to claim 4, and comprising, based on the signals received from the SAS, calculating a position of the distal end.

6.   The method according to claim 4, and comprising presenting the roll angle of the distal end in a coordinate system of the catheter-based tracking system.

7. A magnetic sensor, comprising:

a single axis sensor (SAS), which is configured to, in response to one or more Alternating Current (AC) magnetic fields, generate signals indicative of a direction of a single axis of the SAS in a given coordinate system; and a Hall effect sensor, which is configured to, in response to a Direct Current (DC) magnetic field, generate a signal indicative, in the given coordinate system, of a roll-angle of the SAS about the single axis.

8. The magnetic sensor according to claim 7, wherein the SAS comprises a single coil that is wound to enclose the Hall effect sensor.

9. The magnetic sensor according to claim 8, wherein the SAS and the Hall effect sensor are configured to be fitted at a distal end of a catheter for insertion into an organ of a patient.

10. The magnetic sensor according to claim 7, wherein the SAS is further configured, in response to the to the one or more AC magnetic fields, to generate signals indicative of a position of the SAS in the given coordinate system.

FIG. 1

FIG. 2A

$\alpha = 0°$

$V_H$ is maximal

FIG. 2B

$\alpha = 90°$

$V_H$ is zero

FIG. 3

Normalized Hall Voltage

Roll Angle $\alpha$ [Rad]

```
Image patient
using MRI
system
```
70

```
Track a position and a direction of a
catheter using a catheter-based tracking
system utilizing a SAS in catheter
```
72

```
Sense component of DC magnetic field of MRI
system using Hall-effect sensor in catheter
```
74

```
Register coordinate system of
MRI with coordinate system of
the tracking system
```
75

```
Using tracked direction, sensed component of
DC magnetic field and the registration, calculate
roll-angle of catheter
```
76

```
Present on display direction
and roll-angle of distal end
```
78

```
Align the distal end relative to tissue based on
the calculated position, direction and roll-angle
```
80

```
Perform procedure
```
82

*FIG. 4*

Europäisches
Patentamt

European
Patent Office

Office européen
des brevets

# EUROPEAN SEARCH REPORT

Application Number

EP 19 19 5072

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | US 2018/172420 A1 (HEIN MATTHEW [US] ET AL) 21 June 2018 (2018-06-21)<br>* figures 1,3 *<br>* paragraph [0046] - paragraph [0048] *<br>* paragraph [0052] - paragraph [0055] *<br>* paragraph [0057] - paragraph [0059] *<br>* paragraph [0060] *<br>* paragraph [0067] *<br>----- | 7,10<br><br>1-3,8,9 | INV.<br>A61B34/20<br>A61B5/06<br>G01R33/00 |
| A | US 2005/245811 A1 (SCHEFFLER KLAUS [CH]) 3 November 2005 (2005-11-03)<br>* paragraph [0034] - paragraph [0035]; figure 1 *<br>* paragraph [0043] *<br>----- | 1-3,7-10 | |
| A | US 2014/275957 A1 (LUPOTTI FERMIN A [US]) 18 September 2014 (2014-09-18)<br>* figure 1 *<br>* paragraph [0038] - paragraph [0040] *<br>* paragraph [0039] - paragraph [0040] *<br>----- | 1-3,7-10 | |

TECHNICAL FIELDS
SEARCHED      (IPC)

A61B
G01R

The present search report has been drawn up for all claims

| Place of search | Date of completion of the search | Examiner |
|---|---|---|
| Munich | 21 January 2020 | Roudaut, Tanguy |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 19 5072

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2020

| Patent document cited in search report | Publication date | Patent family member(s) | Publication date |
|---|---|---|---|
| US 2018172420 A1 | 21-06-2018 | NONE | |
| US 2005245811 A1 | 03-11-2005 | NONE | |
| US 2014275957 A1 | 18-09-2014 | NONE | |

EPO FORM P0459

For more details about this annex : see Official Journal of the European Patent Office, No. 12/82

**EP 3 620 127 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100210939 A
- US 20160278746 A
- US 20120143127 A
- US 20030006759 A
- US 5391199 A
- US 6690963 B
- US 6484118 B
- US 6239724 B
- US 6618612 B
- US 6332089 B
- WO 9605768 A
- US 20020065455 A1
- US 20030120150 A1
- US 20040068178 A1